**Europäisches Patentamt**

**European Patent Office**

**Office européen des brevets**

(19)

(11) Publication number : **0 295 112 B1**

(12) **EUROPEAN PATENT SPECIFICATION**

(45) Date of publication of patent specification :
31.08.94 Bulletin 94/35

(51) Int. Cl.⁵ : **G01N 27/416**

(21) Application number : **88305301.9**

(22) Date of filing : **10.06.88**

(54) **Probe for measuring concentration of impurity element in molten metal.**

(30) Priority : **11.06.87 JP 145765/87**

(43) Date of publication of application :
**14.12.88 Bulletin 88/50**

(45) Publication of the grant of the patent :
**31.08.94 Bulletin 94/35**

(84) Designated Contracting States :
**BE DE FR GB**

(56) References cited :
**EP-A- 0 059 222**
**EP-A- 0 208 072**
**US-A- 3 816 269**
**PATENT ABSTRACTS OF JAPAN, vol. 11, no.**
**112 (P-565)(2559), 9 April 1987; &**
**JP-A-61260157**
**PATENT ABSTRACTS OF JAPAN, vol. 10, no.**
**341 (P-547)(2397), 18 November 1986; &**
**JP-A-61142455**
**PATENT ABSTRACTS OF JAPAN, vol. 9, no.**
**227 (P-388)(1950), 13 September 1985; &**
**JP-A-6085361**
**PATENT ABSTRACT OF JAPAN vol. 11, no. 112**
**(P-565)(2559), 9th April 1987; & JP - A - 61 260**
**155**

(73) Proprietor : **Osaka Sanso Kogyo Limited**
**1-14, Miyahara 4-chome**
**Yodogawa-ku Osaka (JP)**
Proprietor : **NIPPON KOKAN KABUSHIKI**
**KAISHA**
**1-2 Marunouchi 1-chome**
**Chiyoda-ku**
**Tokyo 100 (JP)**
Proprietor : **Sasabe, Minoru**
**Daini-Inagehaitsu 20-501**
**1207, Konakadaimachi**
**Chiba-shi Chiba-ken (JP)**

(72) Inventor : **Hamada, Nobuo**
**3-13-17-102 Hon-nakayama**
**Funabashi-shi Chiba-ken (JP)**
Inventor : **Nagatsuka, Toshio**
**4-11-12-101 Tajiri**
**Ichikawa-shi Chiba-ken (JP)**

(74) Representative : **Ritter, Stephen David et al**
**Mathys & Squire**
**10 Fleet Street**
**London EC4Y 1AY (GB)**

## Description

The present invention relates to a probe for measuring the concentration of an impurity element in a molten metal.

As a result of the increase in the number of metallic products available and the improvement in quality thereof, it has recently become important to control the amount of impurity elements contained in molten metals. In the prior art, the most common practice has been to collect a sample for analysis and measure the concentration of an impurity element by instrumental analysis. Such conventional practice suffers, however, from the problem that the measurement cannot be carried out speedily.

Under these circumstances, Patent Publication (Kokai) No. 142455/1986 was proposed as a method which enables fast measurement of the concentration of an impurity element in a molten metal. The proposed method employs a probe which is produced by forming on the surface of a solid electrolyte a coating layer of an oxide of an impurity element as an object of measurement or a compound oxide containing said oxide and any oxide other than it. According to the prior art method, the probe is immersed in a molten metal, and the oxygen potential relative to an equilibrium reaction of the impurity element as the object of measurement and the oxide of said impurity element is measured on the basis of the principle of the oxygen concentration cell to thereby obtain the concentration of the target impurity element.

However, the above-described coating layer suffers from low strength at high temperatures and therefore may be separated on heating before it is immersed in a molten metal or during the immersing. Thus it has been impossible to detect the stable electromotive force of the cell. Accordingly, the prior art involves the problem that the rate of success in measurement is rather low.

Accordingly, it is an object of the present invention to provide a probe for measuring the concentration of an impurity element in a molten metal which is so designed that a coating layer for maintaining the activity of an oxide of an impurity element as an object of measurement at a constant level is firmly and stably held on the surface of a solid electrolyte.

To attain the above-described object, the present inventors added a metallic fluoride (preferably an alkaline earth metal fluoride) to the conventional coating layer of an oxide of an impurity element as the object of measurement or a compound oxide consisting of said oxide and any oxide other than it, the metallic fluoride having high bond strength with respect to said oxide, thereby enabling the coating layer to remain stably on the surface of the solid electrolyte in a molten metal without any fear of separation even if it is subjected to a sudden temperature change. Thus, it becomes possible to obtain a stable electromotive force of the cell at all times, the electromotive force precisely corresponding to the oxygen potential relative to the equilibrium reaction of an impurity element as an object of measurement and an oxide of the target impurity element. Accordingly, it is possible to greatly increase both the rate of success in measurement and the degree of accuracy of measurement and hence to satisfy the operational requirements.

More specifically the present invention provides a probe for measuring the concentration of Si contained as an impurity in a molten metal which comprises a solid electrolyte having oxygen ionic conductivity and having a coating layer provided on its surface, said coating layer comprising $SiO_2$ whereby in use of the probe, an equilibrium reaction $Si + O_2 = SiO_2$ takes place in the vicinity of the coating layer, and a standard electrode for providing a predetermined oxygen potential, characterised in that said coating layer contains $CaF_2$ in an amount such that the ratio of $CaF_2 : SiO_2$ is at least 3:49 whereby the activity of $SiO_2$ is maintained at an approximately constant level.

The solid electrolyte and the standard electrode may be prepared using any materials which are usable to form conventional oxygen sensors.

The above and other objects, features and advantages of the present invention will become apparent from the following description taken in conjunction with the accompanying drawings.

Figs. 1 to 4 show in combination one embodiment of the probe for measuring the concentration of an impurity element in a molten metal according to the present invention, in which:

Fig. 1 schematically shows the arrangement of the probe according to the present invention;

Fig. 2 is a sectional view showing the structure of the probe according to the present invention;

Fig. 3 is a sectional view showing another example in which the solid electrolyte is supported by a member which is additionally provided; and

Fig. 4 shows the arrangement of a probe and devices for detecting and recording electric signals output from the probe.

Figs. 5 to 8 show typical examples of traceforms of electromotive force measured in the Examples, in which:

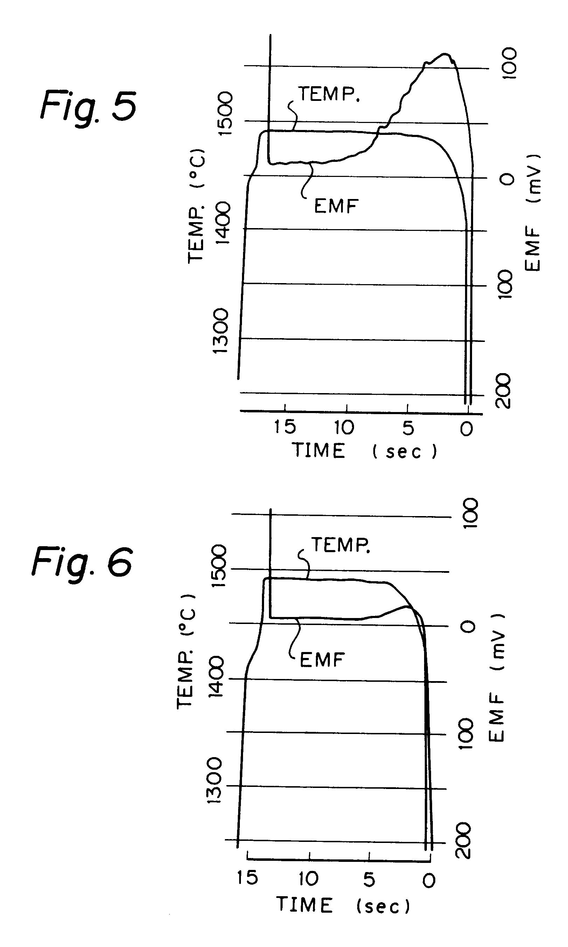

Fig. 5 shows the traceform relative to the prior art probe A;

Fig. 6 shows the traceform relative to the probe B according to the present invention;

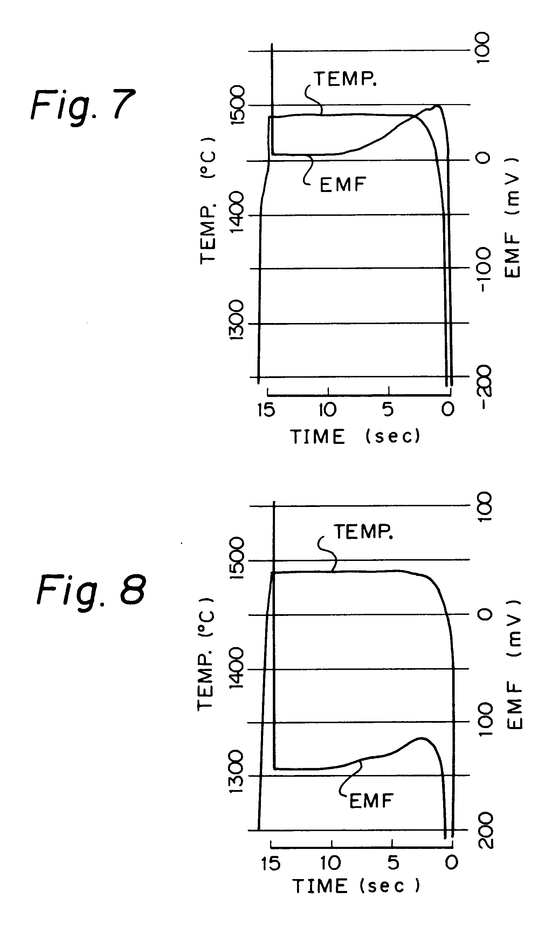

Fig. 7 shows the traceform relative to the probe C according to the present invention; and

Fig. 8 shows the traceform of an oxygen sensor.

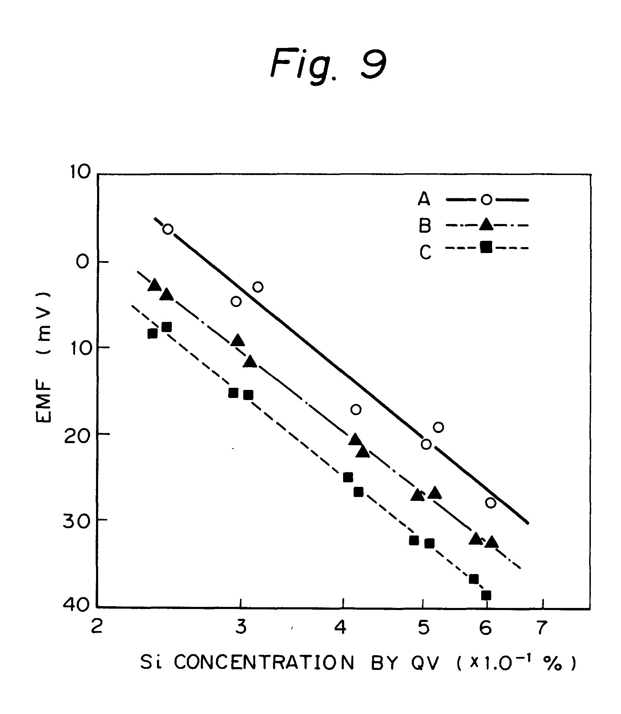

Fig. 9 is a graph showing the relationship between the electromotive force of each of the Si concentration measuring probes measured in Example 1 and the Si concentration obtained by emission spectrography;

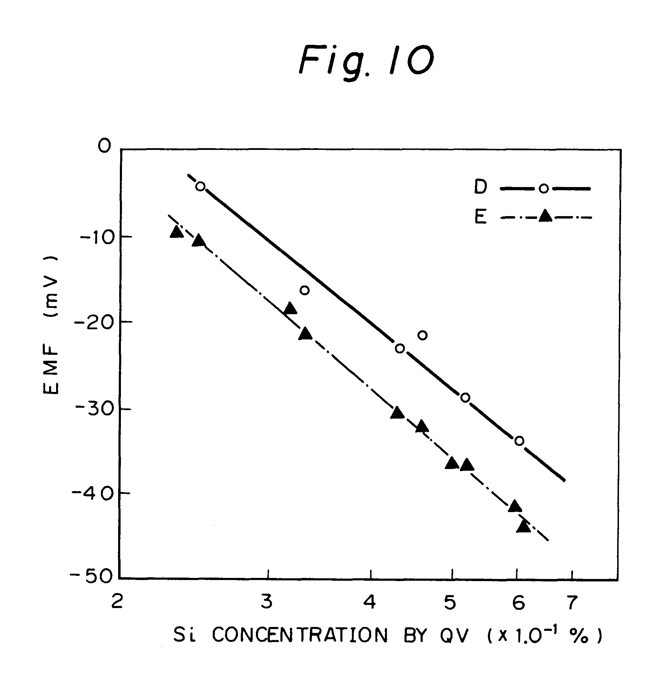

Fig. 10 is a graph showing the relationship between the electromotive force of each of the Si concentration measuring probes measured in Example 2 and the Si concentration obtained by emission spectrography.

The present invention is described hereinunder in detail with reference to the accompanying drawings.

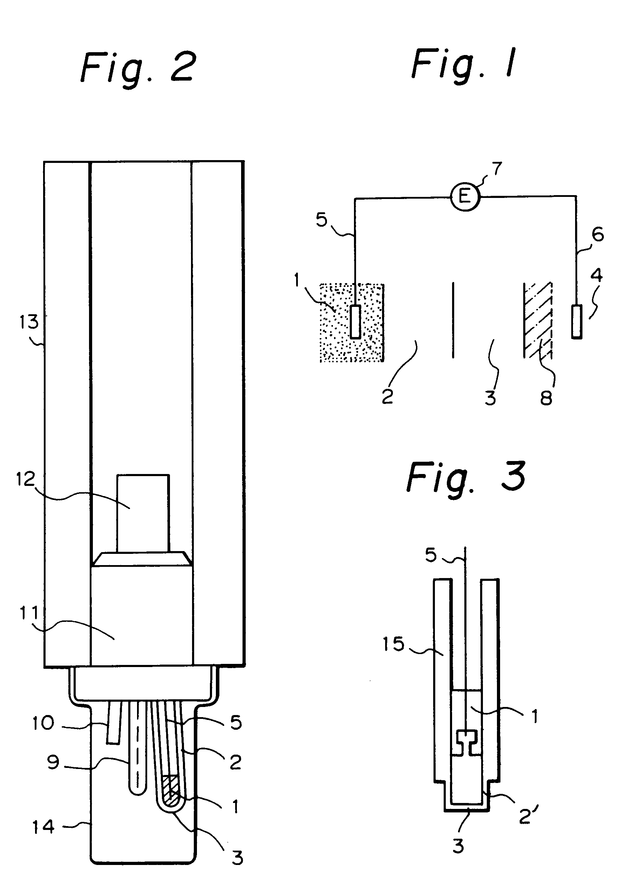

Referring first to Fig. 1, which schematically shows the arrangement of the present invention, the reference numeral 1 denotes a standard electrode, 2 a solid electrolyte, 3 a coating layer, 4 a molten metal, 5 a standard electrode-side lead, 6 a molten metal-side lead, and 7 a potentiometer. The probe according to the present invention has the solid electrolyte 2 covered with the coating layer 3 which acts in a molten metal so as to maintain at a constant level the activity of an oxide of an impurity element as an object of measurement. When the probe is immersed in the molten metal 4, an equilibrium reaction expressed by the following formula takes place in the vicinity of the coating layer 3:

$$M + \frac{x}{2} O_2 = MO_x \quad (1)$$

wherein M is an impurity element as the object of measurement; and O is oxygen.

For simplification, the region in which the reaction of the formula (1) takes place is schematically shown in Fig. 1, as denoted by the reference numeral 8. In this case, the activity of $MO_x$, i.e., $\alpha MO_x$, is 1 or less, but there will be no problem if the activity is constant. Let us assume the activity to be 1 for convenience. The equilibrium constant KM of the reaction (1) is expressed as follows:

$$K_M = \frac{\alpha MO_x}{\alpha_M \cdot Po_2^{\frac{x}{2}}} = \frac{1}{\alpha_M \cdot Po_2^{\frac{x}{2}}} \quad (2)$$

wherein $\alpha M$ is the activity of an impurity element as an object of measurement; and $Po_2$ is the oxygen potential which takes part in the reaction of the formula (1). Since KM is a function of temperature, $\alpha M$ can be obtained simply by measuring the temperature of the molten metal and $Po_2$ with an oxygen sensor. The electromotive force (the indication of the potentiometer 7 shown in Fig. 1) E of an oxygen sensor is generally expressed as follows:

$$E = \frac{RT}{F} \ell n \frac{Po_2 (II) \frac{1}{4} + Pe' \frac{1}{4}}{Po_2 (I) \frac{1}{4} + Pe' \frac{1}{4}} \quad (3)$$

wherein T is the temperature; F is Faraday's constant; R is the gas constant; $Po_2$(I) is the oxygen potential given by the standard electrode; and Pe' is the partial electron conductivity parameter. $Po_2$(II) corresponds to $Po_2^{x/2}$ in the equation (2) and is expressed as follows:

$$Po_2 (II) = \frac{.1}{K_M \cdot \alpha_M} \quad (4)$$

If the equation (4) is substituted in the equation (3), the following expression is obtained:

$$E = \frac{RT}{F} \ell n \frac{(K_M \cdot \alpha_M)^{-\frac{1}{4}} + Pe'^{\frac{1}{4}}}{Po_2 (I) \frac{1}{4} + Pe' \frac{1}{4}} \quad (5)$$

This is solved for $\alpha M$ to obtain the activity of an impurity element in a molten metal as follows:

$$\alpha_M = \frac{[\{(Po_2 (I)^{\frac{1}{4}} + Pe' \frac{1}{4}) \times \exp\frac{FE}{RT}\} - Pe'^{\frac{1}{4}}]^{-4}}{K_M} \quad (6)$$

In general, the relationship between the concentration and activity of a dissolved component in a molten metal is expressed as follows:

$$\log \alpha_M = \log[\%M] + \sum_j \ell_M [\%j] \quad (7)$$

wherein [%M] is the concentration of an impurity element as an object of measurement; [%j] is the concentration of other dissolved components; and $\ell M$ is the interaction auxiliary coefficient. Accordingly, [%M] can be calculated from the equation (7) numerically.

The arrangement of the probe according to the present invention will next be explained more specifically with reference to Fig. 2. The solid electrolyte 2 has the standard electrode 1 disposed therein, the standard electrode-side lead 5 which is led out from the electrode 1, and the coating layer 3 formed on its outside. The solid electrolyte 2, a thermocouple 9 and a molten metal electrode 10 are attached to a housing 11. The electromotive force between the standard electrode 1 and the molten metal electrode 10 and the electromotive force of the thermocouple 9 are supplied to measuring devices (not shown) through a connector 12. The upper part of the housing 11 is fitted into a protective tube 13, while the lower part of the housing 11 is covered with a cap 14.

Fig. 3 shows another example in which the solid electrolyte 2' is supported through a refractory member. More specifically, the standard electrode 1 is disposed inside a refractory tube 15, and one end of the tube 15 is closed with the solid electrolyte 2'. The coating layer 3 is formed on the exposed portion of the solid electrolyte 2.

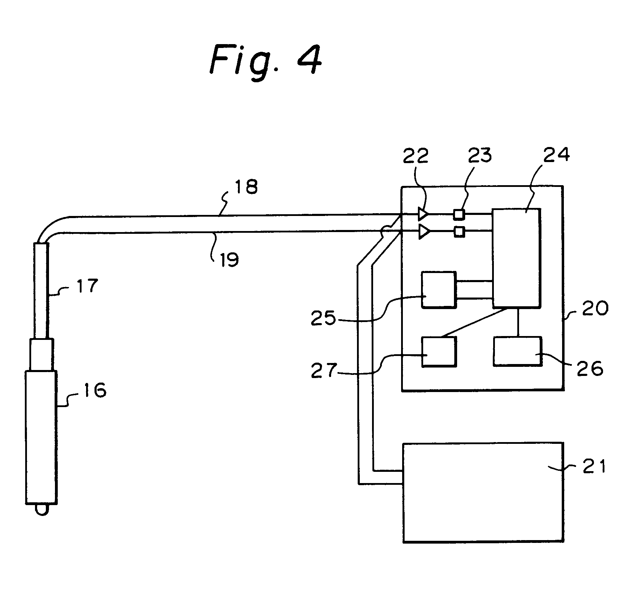

Fig. 4 shows the arrangement of devices employed to convert an electric signal detected by the probe of the present invention into an impurity element concentration. A probe 16 which is attached to a holder 17 is immersed in a molten metal. The signal detected by the probe 16 is passed through a cable extending through the holder 17, and input to both an arithmetic unit 20 and a self-balancing recorder 21 in the form of a temperature signal 18 and an electromotive force signal 19 which corresponds to an impurity element concentration. The self-balancing recorder 21 records on a chart the analog signal from the probe 16 in the form of analogue data. In the arithmetic unit 20, the analogue signal from the probe 16 is amplified in an amplifier 22, converted into a digital signal in an A/D converter 23 and then input to a computer 24 where an impurity element concentration is calculated from the input value according to the above-described equations (6) and (7). The data input to the computer 24 is stored in a memory 25, displayed on a display 26 and also printed out from a printer 27.

The present invention will be explained more specifically below by way of Examples. However, the present invention is in no way restricted to these Examples.

In the Examples, the concentrations of Si in molten iron were measured with probes having the same structure as that shown in Fig. 2.

Materials employed to form the probes are as follows:

Solid electrolyte: $ZrO_2$-8 mol% MgO

Standard electrode: mixture of Cr and $Cr_2O_3$ powder

Standard electrode-side lead: Mo wire having a diameter of 0.29 mm

Molten metal electrode: Mo bar having a diameter of 3 mm

Thermocouple: Type-R

## Example 1

Ingredients shown in Table 1 were mixed together in various ratios, and water was added to the mixtures to prepare three different kinds of coating material in the form of slurries. Each of the coating materials was uniformly coated on the surface of a solid electrolyte in the shape of a tube having one end thereof closed and then dried at room temperature. With these solid electrolytes, three different types of probe were formed.

### Table 1
#### Mixing conditions of coating materials (wt%)

| | | Coating material | | |
|---|---|---|---|---|
| | | A (Prior art) | B | C |
| Components | $SiO_2$ | 58 | 49 | 49 |
| | $CaF_2$ | 0 | 9 | 3 |
| | $MgF_2$ | 0 | 0 | 6 |
| | organic binder | 11 | 11 | 11 |
| | water | 31 | 31 | 31 |

Then, the concentration of Si in molten iron was measured with each of the above-described probes under the following conditions.

Temperature: 1450 to 1500°C
C concentration: 4.5 to 5.0 wt%
P concentration: 0.08 to 0.12 wt%
Mn concentration: 0.15 to 0.2 wt%

Figs. 5 to 8 respectively show typical examples of traceforms of electromotive force obtained in measurement of the Si concentration under the above-described conditions, in which: Fig. 5 shows the traceform relative to the prior art probe A; Fig. 6 shows the traceform relative to the probe B; Fig. 7 shows the traceform relative to the probe C; and Fig. 8 shows the traceform relative to an oxygen sensor. In the case of the prior art probe A containing no metallic fluoride in the coating layer, no stable traceform was obtained because of separation of the coating layer or other kinds of failure and it took about 15 seconds for electromotive force to reach an equilibrium state. In contrast to this, it was possible with the probes B and C to obtain smooth traceforms, and the time required for electromotive force to reach an equilibrium state was about 7 seconds for the probe B and about 10 seconds for the probe C. Thus, the present invention provides a considerable improvement. The comparison of the traceform of the oxygen sensor shown in Fig. 8 and those of the probes A to C reveals that there is a wide difference in terms of electromotive force and hence the Si concentration measuring probes according to the present invention are different from the oxygen sensor.

Fig. 9 shows the relationship between the electromotive force of each of the Si concentration measuring probes in this Example and the Si concentration obtained by emission spectrography. As will be clear from the graph, the prior art A has large variations, whereas the electromotive force of each of the probes B and C according to the present invention has a considerably good linear relation to the Si concentration obtained by emission spectrography.

The rate of success in measurement of the prior art probe A was about 60%, whereas those of the probes B and C were about 98% and 95%, respectively.

## Example 2

Two different kinds of coating layer were formed in accordance with the mixing ratios shown in Table 2 in the same way as in Example 1, and the concentration of Si in molten iron was measured with probes respectively having the resulting coating layers under the following conditions.

Temperature: 1500 to 1550°C
C concentration: 3.8 to 4.5 wt%
P concentration: 0.05 to 0.1 wt%
Mn concentration: 0.10 to 0.17 wt%

### Table 2

#### Mixing conditions of coating materials (wt%)

| | | Coating material | |
|---|---|---|---|
| | | D (Prior art) | E |
| Components | $SiO_2$ | 29 | 29 |
| | $ZrSiO_4$ | 29 | 23 |
| | $CaF_2$ | 0 | 6 |
| | organic binder | 11 | 11 |
| | water | 31 | 31 |

Fig. 10 shows the relationship between the electromotive force of each the Si concentration measuring probes in this Example and the Si concentration obtained by emission spectrography. As will be understood from the graph, the electromotive force of the probe E according to the present invention is linearly related to the Si concentration obtained by emission spectrography, and the probe E has smaller variations than those in the case of the prior art probe D.

As shown in each of the above-described Examples, employment of the probe according to the present invention enables fast measurement of the concentration of an impurity element in a molten metal.

## Claims

1. A probe for measuring the concentration of Si contained as an impurity in a molten metal which comprises a solid electrolyte having oxygen ionic conductivity and having a coating layer provided on its surface, said coating layer comprising $SiO_2$ whereby in use of the probe, an equilibrium reaction $Si + O_2 = SiO_2$ takes place in the vicinity of the coating layer, and a standard electrode for providing a predetermined oxygen potential, characterised in that said coating layer contains $CaF_2$ in an amount such that the ratio of $CaF_2:SiO_2$ is at least 3:49 whereby the activity of $SiO_2$ is maintained at an approximately constant level.

## Patentansprüche

1. Sonde zur Messung der Konzentration von Si, welches als Verunreinigung in einem geschmolzenen Metall enthalten ist, welche eine Feststoffelektrolyten aufweist, der Sauerstoff-Ionenleitfähigkeit hat, und auf ihrer Oberfläche mit einer Beschichtungsschicht versehen ist, wobei die Beschichtungsschicht $SiO_2$ enthält, wodurch im Gebrauch der Sonde eine Gleichgewichtsreaktion $Si + O_2 = SiO_2$ in der Nähe der Beschichtungsschicht stattfindet, und welche eine Standardelektrode zur Bereitstellung eines vorbestimmten Sauerstoffpotentials aufweist, dadurch **gekennzeichnet**, daß die Beschichtungsschicht $CaF_2$ in solcher Menge aufweist, daß das Verhältnis von $CaF_2:SiO_2$ zumindest 3:49 beträgt, wodurch die Aktivität von $SiO_2$ auf einem annähernd konstantem Niveau gehalten wird.

## Revendications

1. Une sonde pour mesurer la concentration de Si contenu sous forme d'impureté dans un métal fondu, laquelle comprend un électrolyte solide ayant une conductivité d'ion oxygène et ayant une couche de revêtement prévue à sa surface, ladite couche de revêtement comprenant le $SiO_2$, grâce à quoi en cours d'utilisation de la sonde, une réaction d'équilibre $Si+O_2 = SiO_2$ a lieu au voisinage de la couche de revêtement et une électrode normalisée pour fournir un potentiel prédéterminé d'oxygène, caractérisée en ce que ladite couche de revêtement contient du $CaF_2$ en une quantité telle que le rapport de $CaF_2 : SiO_2$ est d'au moins 3:49, grâce à quoi l'activité de $SiO_2$ est maintenue à un niveau approximativement constant.

# Fig. 2

# Fig. I

# Fig. 3

# Fig. 4

Fig. 5

Fig. 6

*Fig. 7*

*Fig. 8*

10

# Fig. 9

# Fig. 10